# EUROPEAN PATENT APPLICATION

(11) **EP 4 614 142 A1**
(43) Date of publication of application: **10.09.2025**
(21) Application number: 24895158.4
(22) Date of filing: 22.03.2024
(51) Int. Cl.: G01N 33/00, G01N 1/28, G01N 1/34

(54) **MULTI-FLOW-PATH PROCESS GAS ONLINE ANALYSIS AND PRETREATMENT DEVICE**

(30) Priority: 29.11.2023 CN 202323261238 U
(71) Applicant: Hangzhou Emust Technology Co., Ltd., Hangzhou, Zhejiang 311305 (CN)
(72) Inventor: LI, Anlin, Hangzhou, Zhejiang 311305 (CN); ZHANG, Tengfei, Hangzhou, Zhejiang 311305 (CN); YANG, Huan, Hangzhou, Zhejiang 311305 (CN)
(74) Representative: Mohun, Stephen John
(86) International application number: PCT/CN2024/083336
(87) International publication number: WO 2025/112246

(57) **Abstract**

A multi-flow-path process gas online analysis pretreatment apparatus is provided. A two-position three-way valve can switch gas flow direction and can switch between communication between main gas exhaust pipe and gas intake path and communication between main testing sample gas import path and gas intake path. A multi-channel switching valve switches between communications between different gas intake paths and main testing sample gas import path according to different requirements. A primary pressure reducing valve adjusts pressure of testing sample gas and depressurize a gas whose pressure is higher than a preset value. An impurity filter removes mechanical impurities from a gas under test. A secondary pressure reducing valve performs secondary pressure regulation on the gas depressurized by the primary pressure reducing valve. A three-way filter removes mechanical impurities, water vapor, and petroleum hydrocarbons from the gas under test, preventing impurities from entering key devices or elements. According to the present disclosure, timeliness of monitoring data is effectively improved, errors generated during manual sampling, transportation, and analysis are reduced, analysis data is timely and reliable, analysis cost of process gas is lowered, and efficiency is improved.

## Description

### TECHNICAL FIELD

The present disclosure relates to the technical field of gas analysis, and in particular, to a multi-flow-path process gas online analysis pretreatment apparatus.

### BACKGROUND

In a chemical production process, complex material transformation and reaction processes are involved, and various parameters are required to be monitored and controlled in real time. Through online analysis, key indexes in the production process can be obtained in real time. With real-time data, operators can adjust operating conditions in a timely manner, which optimizes the production process and ensures product quality and economic benefits. However, there are a lot of uncertainties and non-linear factors in the industrial production process. For example, chemical changes, physical changes, and the like often exist in the production process. Very harsh process conditions or production environments such as high temperature, low temperature, high pressure, and high-density dust may exist in some product processes. Sometimes there are even flammable, explosive, corrosive, or toxic materials, which requires relatively high requirements for safety. Therefore, pretreatment for gas samples plays an important role in online analysis, which can effectively protect a back-end analysis instrument and prolong a service life thereof.

In the prior art, a multi-flow-path automatic switching online analysis system includes sample flow paths, purge gas intake pipes, a standard gas container, a pressure reducing valve, and a filter. At least two sample flow paths are connected in parallel through three-way valves. One purge gas intake pipe is arranged between each sample flow path and the three-way valve. The three-way valve of the top layer is sequentially communicated with the pressure reducing valve and the filter through a pipeline. The pipeline between the pressure reducing valve and the filter is communicated with the standard gas container through a three-way selector valve. The filter is communicated with a sample inlet end of a chromatographic instrument. Samples in the multiple paths can be automatically and quickly switched at the same time. A purge gas source is configured during switching the samples in the respective paths. Disadvantages are as follows.
1. When a sample is imported into the flow path of each layer, three-way solenoid valves in the flow paths of other layers are required to be actuated to corresponding valve positions. Once one of these three-way solenoid valves fails, the system cannot operate normally. More flow paths can lead to a higher failure rate.
2. There is a "dead volume" problem in both a pipeline between two ball valves that are located on a pipeline between each sample flow path and the purge gas intake pipe and a pipeline between the ball valve and the three-way solenoid valve.

In the prior art, a process gas online analysis pretreatment system is further disclosed, including: one or more gas intake paths, one or more gas intake main valves, a primary pressure reducing valve, an impurity filter, a secondary pressure reducing valve, a three-way filter, an adjustable flow meter for branch, a branch check valve, an unloading valve, an automatic drain valve, a main exhaust pressure gauge, a multi-channel switching valve, a three-way reversing valve, an adjustable flow meter for main path, a main path check valve, an adjustable flow meter for instrument, a programmable logic controller (PLC), and a control computer. With the system, pressure inside a pipe can be known in time to facilitate maintenance and troubleshooting, and the problem of "dead volume" in the pipelines during gas path replacement is solved, ensuring accuracy of gas testing. Furthermore, testing requirements for various high-pressure process gases can be met, and influences caused by main pipeline gas intake pressure fluctuations on testing results can be eliminated. In addition, the process gas can be prevented from condensing into liquid in the main gas exhaust pipe, where liquid accumulation may cause "liquid seal" in the pipeline and cause exhaust blockage. Disadvantages are as follows.

For gas material pipelines required to be tested, each gas intake pipeline is individually equipped with one pretreatment device. As the quantity of testing gas paths increases, the quantity of pretreatment devices increases, resulting in relatively high equipment cost.

### SUMMARY

In order to overcome the defects in the prior art, a multi-flow-path process gas online analysis pretreatment apparatus is provided, including two or more gas intake paths, two or more main gas intake valves, and two or more two-position three-way valves, and further including a main gas exhaust pipe, a multi-channel switching valve, a primary pressure reducing valve, an impurity filter, a secondary pressure reducing valve, a three-way filter, an adjustable flow meter, a standard gas intake valve, a PLC, and a control computer.

The gas intake paths, the main gas intake valves, and the two-position three-way valves are sequentially connected, and the main gas intake valves control opening and closing of the gas intake paths.

The two-position three-way valve is able to switch a gas flow direction, and is configured to switch between communication between the main gas exhaust pipe and the gas intake path and communication between a main testing sample gas import path and the gas intake path. The main testing sample gas import path includes the multi-channel switching valve, the primary pressure reducing valve, the impurity filter, the secondary pressure reducing valve, the three-way filter, and the adjustable flow meter that are sequentially connected.

The multi-channel switching valve is configured to switch between communications between different gas intake paths and the main testing sample gas import path according to different requirements.

The primary pressure reducing valve is configured to adjust a pressure of a testing sample gas and depressurize a gas whose pressure is higher than a preset value.

The impurity filter is configured to remove mechanical impurities from a gas under test.

The secondary pressure reducing valve is configured to perform secondary pressure regulation on the gas depressurized by the primary pressure reducing valve, an accuracy of the secondary pressure reducing valve being higher than that of the primary pressure reducing valve.

The three-way filter is configured to remove mechanical impurities, water vapor, and petroleum hydrocarbons from the gas under test, and prevent impurities from entering key devices or elements.

The adjustable flow meter is a measuring meter for measuring a gas flow rate, and is configured to adjust a flow rate of the gas under test and display real-time flow rate data.

The standard gas intake valve is configured to control opening and closing of a standard gas intake path.

The PLC has an input/output (I/O) signal input point connected to the control computer, and an I/O signal output point connected to a circuit control part for the two-position three-way valves, the multi-channel switching valve, and the standard gas intake valve.

Preferably, the main gas intake valves are butterfly valves, ball valves, needle valves, or stop valves.

Preferably, the two-position three-way valves are solenoid-driven or pneumatically-driven.

Preferably, the multi-channel switching valve is solenoid-driven or pneumatically-driven.

Preferably, the primary pressure reducing valve is a lever-type pressure reducing valve, a diaphragm-type pressure reducing valve, a piston-type pressure reducing valve, or a bellows-type pressure reducing valve.

Preferably, the impurity filter is a mesh filter, a sintered metal filter, or a magnetic filter.

Preferably, the secondary pressure reducing valve is a diaphragm-type pressure reducing valve, a piston-type pressure reducing valve, or a bellows-type pressure reducing valve.

Preferably, a filter cartridge of the three-way filter is a sintered cartridge, an activated carbon cartridge, a polypropylene (PP) cotton cartridge, a ceramic cartridge, or a polypropylene fiber cartridge.

Preferably, the adjustable flow meter is a differential pressure flow meter, a rotor flow meter, or a throttle flow meter.

Preferably, the standard gas intake valve is a butterfly valve, a ball valve, a needle valve, or a stop valve.

Compared with the prior art, the present disclosure achieves at least the following beneficial effects.
(1) The gas intake path uses a two-position three-way valve to switch a flow direction of a process gas. The process gas in each gas path uses a same pretreatment apparatus located on the main testing sample gas import path, which simplifies pipe valves and reduces the overall equipment cost.
(2) One main testing gas path is used, which simplifies multiple channels of pretreatment units into one set, reduces connection points on respective pipelines, and decreases a failure rate.
(3) By maintaining Port A and Port B of the two-position three-way valve connected, the process gas is discharged to the main gas exhaust pipe through the two-position three-way valve on the gas intake path, which shortens a distance between a gas material and a back-end instrument and reduces replacement time.
(4) The problem of "dead volume" in the pipeline during the gas path replacement is solved by a combination of the multi-channel switching valve and the two-position three-way valves, ensuring accuracy of gas testing.
(5) The design of two-stage pressure reducing valves used in the main gas path can meet the testing requirements for various high-pressure process gases and can also eliminate the influences caused by the main pipeline gas intake pressure fluctuations on the testing results.

### BRIEF DESCRIPTION OF THE DRAWINGS

FIG. 1 is a schematic structural diagram of a multi-flow-path process gas online analysis pretreatment apparatus according to an embodiment of the present disclosure;
FIG. 2 is a schematic diagram of flow channels of a multi-channel switching valve of a multi-flow-path process gas online analysis pretreatment apparatus according to an embodiment of the present disclosure; and
FIG. 3 is a schematic diagram of a control logic of a multi-flow-path process gas online analysis pretreatment apparatus according to an embodiment of the present disclosure.

Numerical references 1, 2, 3, 4, 5 and 6 indicate gas intake paths; 101, 201, 301, 401, 501 and 601 indicate gas intake path main valves; 102, 202, 302, 402, 502 and 602 indicate two-position three-way valves; 01 indicates a main gas exhaust pipe; 02 indicates a multi-channel switching valve; 03 indicates a primary pressure reducing valve; 04 indicates an impurity filter; 05 indicates a secondary pressure reducing valve; 06 indicates a three-way filter; 07 indicates an adjustable flow meter; 08 indicates a standard gas intake valve; 7 indicates a standard gas intake path; 8 indicates an instrument gas path; 09 indicatesa Programmable Logic Controller (PLC); 10 indicates a control computer.

### DETAILED DESCRIPTION

In order to facilitate understanding of the present disclosure, the present disclosure is described comprehensively below with reference to the relevant drawings. Preferred embodiments of the present disclosure are provided in conjunction with the accompanying drawings. Nevertheless, the present disclosure may be embodied in various different forms and is not limited to the embodiments described herein. Rather, these embodiments are provided for thorough and comprehensive understanding of the present disclosure.

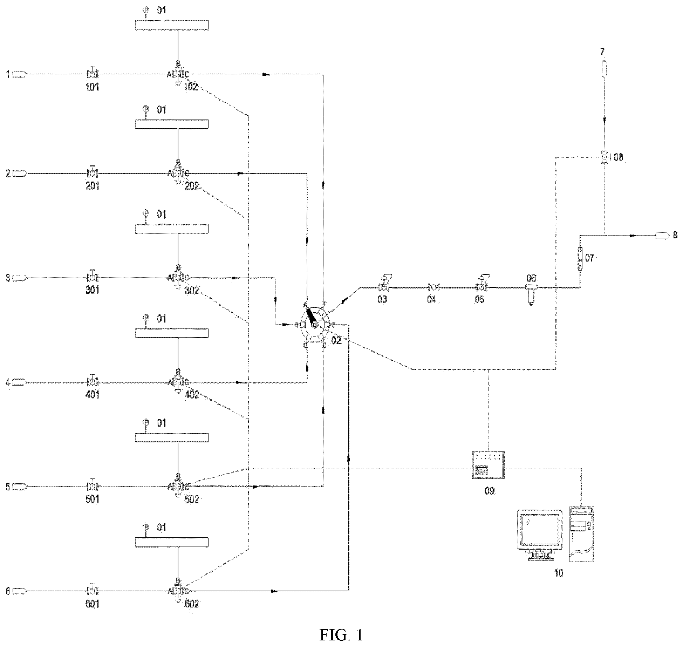

Reference can be made to FIG. 1. A multi-flow-path process gas online analysis pretreatment apparatus is provided according to an embodiment. As shown in FIG. 1, there are 6 flow paths. The apparatus includes: gas intake paths 1, 2, 3, 4, 5 and 6; gas intake path main valves 101, 201, 301, 401, 501 and 601; two-position three-way valves 102, 202, 302, 402, 502 and 602; a main gas exhaust pipe 01; a multi-channel switching valve 02; a primary pressure reducing valve 03; an impurity filter 04; a secondary pressure reducing valve 05; a three-way filter 06; an adjustable flow meter 07; a standard gas intake valve 08; a standard gas intake path 7; an instrument gas path 8; a PLC 09; and a control computer 10.

According to a specific embodiment, in the process gas online analysis pretreatment apparatus, the gas intake paths 1, 2, 3, 4, 5 and 6 are connected to one ends of the gas intake path main valves 101, 201, 301, 401, 501 and 601, the other ends of the gas intake path main valves 101, 201, 301, 401, 501 and 601 are connected to Ports A of the two-position three-way valves 102, 202, 302, 402, 502 and 602. Ports B of the two-position three-way valves 102, 202, 302, 402, 502 and 602 are connected to the main gas exhaust pipe 01. Ports C of the two-position three-way valves 102, 202, 302, 402, 502 and 602 are respectively connected to Port A, Port B, Port C, Port D, Port E, and Port F of the multi-channel switching valve 02. Port G of the multi-channel switching valve 02 is connected to one end of the primary pressure reducing valve 03. The other end of the primary pressure reducing valve 03 is connected to one end of the impurity filter 04. The other end of the impurity filter 04 is connected to one end of the secondary pressure reducing valve 05. The other end of the secondary pressure reducing valve 05 is connected to the three-way filter 06. Another end of the three-way filter 06 is connected to the adjustable flow meter 07. The other end of the adjustable flow meter 07 is connected to a port of the instrument gas path 8. One end of the standard gas intake valve 08 is connected to a port of the standard gas intake path 7, and the other end of the standard gas intake valve 08 is connected to the port of the instrument gas path 8.

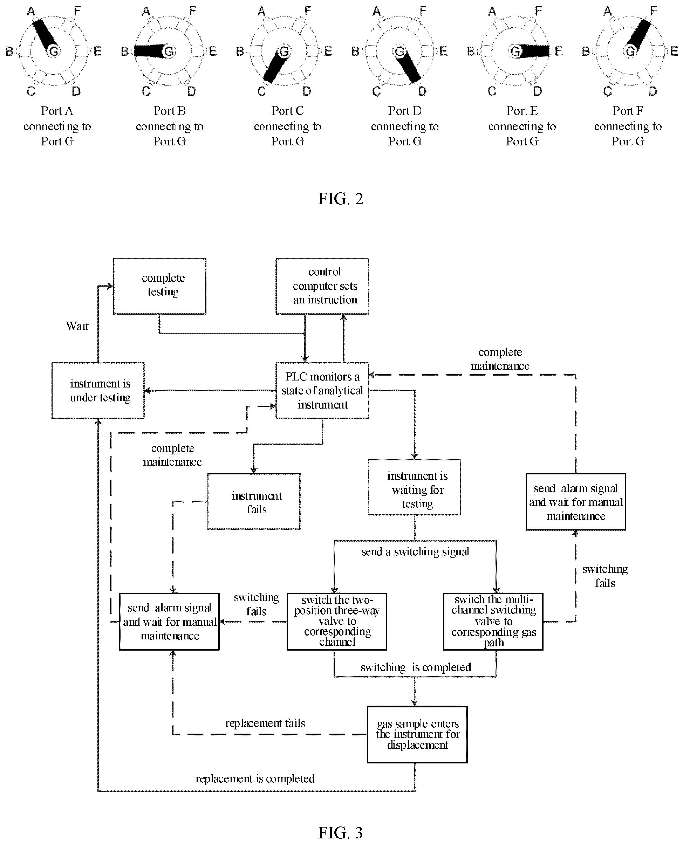

The two-position three-way valves 102, 202, 302, 402, 502 and 602 control the gas intake paths 1, 2, 3, 4, 5 and 6 to switch between the main gas exhaust pipe 01 and the multi-channel switching valve 02. Referring to FIG. 2, the multi-channel switching valve 02 is a switching valve having 6 inlets and 1 outlet, which controls switching among respective connections between the gas intake paths 1, 2, 3, 4, 5 and 6 and the primary pressure reducing valve 03.

The PLC 09 has an Input/Output (I/O) control signal port connected to control ports of the two-position three-way valves 102, 202, 302, 402, 502 and 602, and an I/O control signal port connected to the multi-channel switching valve 02. More preferably, the PLC 09 has an I/O control signal port of connected to an I/O control signal port of an analytical instrument. In response to instructions sent by an upper computer control software deployed on the control computer 10 to the PLC 09, the PLC 09 completes control processes such as gas intake path switching among the gas intake paths 1, 2, 3, 4, 5 and 6, control of time spent on gas replacement, and testing and analyzing. In addition, the PLC 09 can receive a state signal from the I/O control signal port of the analytical instrument, convert the state signal into a digital signal that can be recognized by the control computer 10, and then deliver the digital signal to the upper computer control software for determination, the digital signal serving as an operation parameter of a control instruction.

FIG. 3 schematically illustrates a control logic of a process gas online analysis pretreatment apparatus provided in the present disclosure.

According to a specific embodiment, in an initial state of operation of the aforesaid apparatus, a gas intake path main valve to be analyzed is in a normally open state, and Port A and Port B of the two-position three-way valve are connected. A sample gas under test sequentially passes through the gas intake path main valve and the two-position three-way valve, and then enters the main gas exhaust pipe 01, achieving real-time continuous replacement of the sample gas. Hence, the sample gas under test can be timely imported to back-end, ensuring technicality and accuracy of analysis.

In the initial state of operation of the aforesaid apparatus, Port A and Port B of the two-position three-way valve 03 are connected, and the multi-channel switching valve 02 may be in any state. The PLC 09 monitors the state signal sent by the analytical instrument. In a case that the state signal indicates ready, the PLC 09 reports an instrument ready signal to the control computer 10, the control computer 10 sends a switching signal to the PLC 09 according to an analysis requirement manually set in the upper computer control software, and the PLC 09 controls the two-position three-way valve to switch to connection between Port A and Port C and controls the multi-channel switching valve 02 to complete switching of a corresponding gas intake path. Here, the sample gas under test sequentially passes through the gas intake path main valve, the two-position three-way valve, the multi-channel switching valve 02, the primary pressure reducing valve 03, the impurity filter 04, the secondary pressure reducing valve 05, the three-way filter 06, and the adjustable flow meter 07, and finally enters the instrument gas path 8. After high-pressure sample gas under test passes through the primary pressure reducing valve 03, the pressure of the sample gas drops to an adjustable range of the secondary pressure reducing valve 05, which can meet testing requirements for various high-pressure process gases. The impurity filter 04 is arranged prior to the secondary pressure reducing valve 05 to protect a back-end precision instrument from being damaged by mechanical impurity particles.

After the analytical instrument completes analysis and testing on the sample gas under test, the instrument ready signal is reported again to the control computer 10 through the PLC 09, and the control computer 10 performs a next round of control again according to the analysis requirement manually set in the upper computer control software. By repeating the control logic cyclically, one analytical instrument can perform tests on multiple gas paths, effectively improving timeliness of data monitoring. In addition, errors generated during manual sampling, transportation, and analysis can be eliminated. Due to timely and reliable analysis data, analysis cost of process gas is reduced and the efficiency is improved.

The above-described embodiments are merely used to help understanding the solution of the present disclosure and the core idea thereof. It should be noted that for those of ordinary skill in the art, several improvements and modifications can be made to the present disclosure without departing from the principle of the present disclosure, and these improvements and modifications also fall within the protection scope defined by the claims of the present disclosure.

The above description of the disclosed embodiments enables those skilled in the art to implement or use the present disclosure. Various modifications to these embodiments shall be readily apparent to those skilled in the art, and the generic principles defined herein may be practiced in other embodiments without departing from the spirit or scope of the present disclosure. Therefore, the present disclosure is not intended to be limited to the embodiments disclosed herein but is to be in line with the broadest scope consistent with the principles and novel features disclosed herein.

## Claims

1. A multi-flow-path process gas online analysis pretreatment apparatus, **characterized by** comprising two or more gas intake paths, two or more main gas intake valves, and two or more two-position three-way valves, and further comprising a main gas exhaust pipe, a multi-channel switching valve, a primary pressure reducing valve, an impurity filter, a secondary pressure reducing valve, a three-way filter, an adjustable flow meter, a standard gas intake valve, a programmable logic controller, PLC, and a control computer, wherein
the gas intake paths, the main gas intake valves, and the two-position three-way valves are sequentially connected, the main gas intake valves controlling opening and closing of the gas intake paths;
the two-position three-way valve, which is able to switch a gas flow direction, is configured to switch between communication between the main gas exhaust pipe and the gas intake path and communication between a main testing sample gas import path and the gas intake path, wherein the main testing sample gas import path comprises the multi-channel switching valve, the primary pressure reducing valve, the impurity filter, the secondary pressure reducing valve, the three-way filter, and the adjustable flow meter that are sequentially connected;
the multi-channel switching valve is configured to switch between communications between different gas intake paths and the main testing sample gas import path according to different requirements;
the primary pressure reducing valve is configured to adjust a pressure of a testing sample gas and depressurize a gas whose pressure is higher than a preset value;
the impurity filter is configured to remove mechanical impurities from a gas under test; the secondary pressure reducing valve is configured to perform secondary pressure regulation on the gas depressurized by the primary pressure reducing valve, an accuracy of the secondary pressure reducing valve being higher than that of the primary pressure reducing valve;
the three-way filter is configured to remove mechanical impurities, water vapor, and petroleum hydrocarbons from the gas under test, and prevent impurities from entering key devices or elements;
the adjustable flow meter is a measuring meter for measuring a gas flow rate, and is configured to adjust a flow rate of the gas under test and display real-time flow rate data;
the standard gas intake valve is configured to control opening and closing of a standard gas intake path; and
the PLC comprises an input/output, I/O, signal input point connected to the control computer, and an I/O signal output point connected to a circuit control part for the two-position three-way valves, the multi-channel switching valve, and the standard gas intake valve.

2. The multi-flow-path process gas online analysis pretreatment apparatus according to claim 1, wherein the main gas intake valves are butterfly valves, ball valves, needle valves, or stop valves.

3. The multi-flow-path process gas online analysis pretreatment apparatus according to claim 1, wherein the two-position three-way valves are solenoid-driven or pneumatically-driven.

4. The multi-flow-path process gas online analysis pretreatment apparatus according to claim 1, wherein the multi-channel switching valve is solenoid-driven or pneumatically-driven.

5. The multi-flow-path process gas online analysis pretreatment apparatus according to claim 1, wherein the primary pressure reducing valve is a lever-type pressure reducing valve, a diaphragm-type pressure reducing valve, a piston-type pressure reducing valve, or a bellows-type pressure reducing valve.

6. The multi-flow-path process gas online analysis pretreatment apparatus according to claim 1, wherein the impurity filter is a mesh filter, a sintered metal filter, or a magnetic filter.

7. The multi-flow-path process gas online analysis pretreatment apparatus according to claim 1, wherein the secondary pressure reducing valve is a diaphragm-type pressure reducing valve, a piston-type pressure reducing valve, or a bellows-type pressure reducing valve.

8. The multi-flow-path process gas online analysis pretreatment apparatus according to claim 1, wherein a filter cartridge of the three-way filter is a sintered cartridge, an activated carbon cartridge, a polypropylene, PP, cotton cartridge, a ceramic cartridge, or a polypropylene fiber cartridge.

9. The multi-flow-path process gas online analysis pretreatment apparatus according to claim 1, wherein the adjustable flow meter is a differential pressure flow meter, a rotor flow meter, or a throttle flow meter.

10. The multi-flow-path process gas online analysis pretreatment apparatus according to claim 1, wherein the standard gas intake valve is a butterfly valve, a ball valve, a needle valve, or a stop valve.
